(19) Europäisches Patentamt
European Patent Office
Office européen des brevets

(11) Veröffentlichungsnummer: **0 209 022**
**B1**

(12) # EUROPÄISCHE PATENTSCHRIFT

(45) Veröffentlichungstag der Patentschrift: **18.10.89**

(51) Int. Cl.⁴: **C07D 495/04**, A61K 31/55
// (C07D495/04, 223:00, 333:00)

(21) Anmeldenummer: **86109187.4**

(22) Anmeldetag: **04.07.86**

(54) **4-Substituierte 10-Cyanmethylen-thieno[4,3-e]-benzo-azepine.**

(30) Priorität: **11.07.85 DE 3524744**

(43) Veröffentlichungstag der Anmeldung:
**21.01.87 Patentblatt 87/4**

(45) Bekanntmachung des Hinweises auf die Patenterteilung:
**18.10.89 Patentblatt 89/42**

(84) Benannte Vertragsstaaten:
**AT BE CH DE FR GB IT LI NL SE**

(56) Entgegenhaltungen:
**EP-A- 0 001 401**
**DE-A- 2 918 778**
**DE-A- 3 037 971**

**Medical Chemistry: Berger, Part I, Willy-Interscience, 1970, Seite 77**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen(DE)**

(72) Erfinder: **Steiner, Gerd, Dr., Oberer Waldweg 1, D-6719 Kirchheim(DE)**
Erfinder: **Teschendorf, Hans-Jürgen, Dr., Georg-Nuss-Strasse 5, D-6724 Dudenhofen(DE)**
Erfinder: **Unger, Liliane, Dr., Waltraudenstrasse 14, D-6700 Ludwigshafen(DE)**

**EP 0 209 022 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft in 4-Stellung substituierte 10-Cyanmethylenthieno[4,3-e]-benzo-azepine, Verfahren zu ihrer Herstellung und ihre Anwendung als Arzneimittel, die als Sedativa, Hypnotika, Tranquilizer, Muskelrelaxantien, Neuroleptika oder Antiparkinsonmittel verwendet werden können.

Es ist bekannt, daß tricyclische Ringsysteme mit einer Dibenzo-Struktur zu einem zentralen heterocyclischen 7-Ring, der gegebenenfalls einen basischen Seitenrest, wie beispielsweise einen N-Methylpiperazinrest aufweist, neuroleptische Wirkungen aufweisen können. Solche Tricyclen sind beispielsweise N-Methylpiperazin-Derivate von Dibenzo[b,e,][1,4]-diazepinen (Clozapine), Dibenzo[b,f][1,4]-thiazepinen (Clotiapine), Dibenzo[b,f][1,4]-oxazepinen (Loxapine) oder Morphantridinen (Perlapine), wie beispielsweise aus der Zusammenfassung von J. Schmutz in der Arzneimittelforschung 25, 712-720 (1975) hervorgeht.

In der DE-OS 29 18 778 und der DE-OS 30 37 971 werden 6-substituierte 11-Alkylen-morphantridine bzw. 5-substituierte-9-Cyanmethylen-dithieno[3,4-b:4'.3'-e]azepine mit wertvollen pharmakologischen Eigenschaften beschrieben.

Es wurde nun gefunden, daß 4-substituierte 10-Cyanmethylen-thieno[4,3-e]-benzo-azepine der Formel I

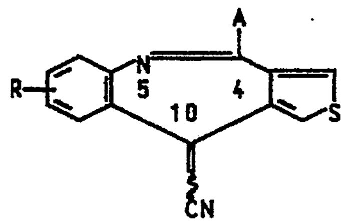

I,

in der

R ein Wasserstoff- oder Halogenatom, einen Alkylrest mit 1 bis 3 C-Atomen, Trifluormethyl oder einen Alkoxyrest mit 1 bis 3 C-Atomen bedeutet, und A für einen Aminorest $-NR^1R^2$ steht, in dem $R^1$ und $R^2$ zusammen mit dem sie verbindenden Stickstoffatom einen 5- bis 7-gliedrigen gesättigten Ring bedeuten, der gegebenenfalls als weiteres Heteroatom ein Stickstoff-oder Sauerstoffatom enthält, wobei ein zusätzlich vorhandenes Stickstoffatom gegebenenfalls durch einen Alkylrest mit 1 bis 3 C-Atomen, einen Hydroxyalkylrest mit 2 bis 3 C-Atomen, einen Alkoxyalkylrest mit 2 bis 3 C-Atomen im Alkyl- oder Alkoxyrest, einen Cycloalkyl- oder Cycloalkylmethylrest mit 3 bis 7 C-Atomen im Cycloalkylring, einen Alkinylrest mit 2 bis 5 C-Atomen und gegebenenfalls zusätzlich durch Sauerstoff in Form eines N-Oxids substituiert ist, oder A einen Aminorest $-NHR^3$, in dem $R^3$ einen Aminoalkylrest mit 2 bis 7 C-Atomen bedeutet, wobei das Aminstickstoffatom gegebenenfalls durch einen niederen Alkylrest mit 1 bis 5 C-Atomen substituiert oder Bestandteil eines 5- bis 7-gliedrigen gesättigten Ringes ist, der gegebenenfalls ein Stickstoff- oder Sauerstoffatom als weiteres Heteroatom enthält, wobei ein vorhandenes Stickstoffatom durch einen niederen Alkylrest mit 1 bis 3 C-Atomen oder einen Hydroxyalkylrest mit 2 bis 3 C-Atomen substituiert sein kann, bedeutet, und ihre physiologisch verträglichen Säureadditionssalze wertvolle pharmakologische Eigenschaften aufweisen.

Für den Rest R kommen insbesondere folgende Bedeutungen in Betracht:
Wasserstoff, Fluor, Chlor, Methyl, Trifluormethyl und Methoxy.

Als Aminreste $-NR^1R^2$ für A sind vorzugsweise Piperazinyl-, Homopiperazinyl-, Piperidinyl- und Morpholinylreste zu nennen.

Besonders bevorzugte Reste $-NR^1R^2$ sind der 4-Methyl-piperazinyl-, 4-Methyl-4-oxy-piperazinyl-, 4-Ethyl-piperazinyl- und der N-Methyl-homopiperazinyl-Rest.

Im Aminrest $-NHR^3$ sind für $R^3$ der 2-Dimethylamino-ethyl- und der 2-Piperidin-1-yl-ethyl-Rest hervorzuheben.

Es sei darauf hingewiesen, daß die erfindungsgemäßen Verbindungen der Formel I als cis-trans-Isomere Ia und b vorliegen.

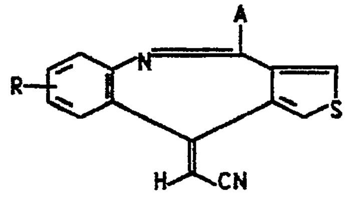

Ia

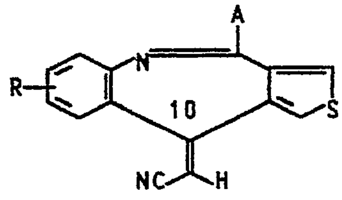

Ib

Die cis-trans-Isomeren können beispielsweise durch fraktionierte Kristallisation oder durch Säulenchromatographie getrennt werden.

Die Zuordnung der einzelnen Isomeren erfolgt beispielsweise durch Röntgenstrukturanalyse, wie aus den Beispielen hervorgeht.

Aufgrund der oben genannten Bedeutungen sind die folgenden Verbindungen als besonders bevorzugt und wirksam zu nennen:

cis,trans-10-Cyanmethylen-4-(4-methyl-piperazin-1-yl)-thieno[4,3-e]benzo-azepin
cis-10-Cyanmethylen-4-(4-methyl-piperazin-1-yl)-thieno[4,3-e]benzo-azepin
trans-10-Cyanmethylen-4-(4-methyl-piperazin-1-yl)-thieno[4,3-e]benzo-azepin
cis,trans-7-Chlor-10-cyanmethylen-4-(4-methyl-piperazin-1-yl)-thieno[4,3-e]benzo-azepin
cis-7-Chlor-10-cyanmethylen-4-(4-methyl-piperazin-1-yl)-thieno[4,3-e]benzo-azepin
trans-7-Chlor-10-cyanmethylen-4-(4-methyl-piperazin-1-yl)-thieno[4,3-e]benzo-azepin
cis, trans-7-Fluor-10-cyanmethylen-4-(4-methyl-piperazin-1-yl)-thieno[4,3-e]-benzo-azepin
cis, trans-7-Methyl-10-cyanmethylen-4-(4-methyl-piperazin-1-yl)-thieno[4,3-e]benzo-azepin
cis, trans-7-Trifluormethyl-10-cyanmethylen-4-(4-methyl-piperazin-1-yl)-thieno[4,3-e]benzo-azepin
cis, trans-7-Methoxy-10-cyanmethylen-4-(4-methyl-piperazin-1-yl)-thieno[4,3-e]benzo-azepin
cis, trans-10-cyanmethylen-4-piperazin-1-yl-thieno[4,3-e]benzo-azepin

Wie die Ausführungsbeispiele zeigen, kann im Einzelfall die Trennung in das cis- und trans-Isomere ohne allzu großen Aufwand vorgenommen werden.

Die erfindungsgemäßen Verbindungen der Formel I werden hergestellt, indem man eine Verbindung der Formel II

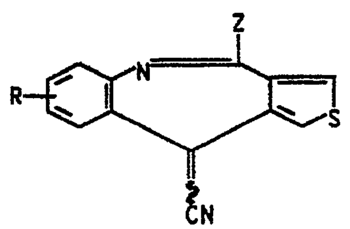

II,

in der R die angegebenen Bedeutungen hat und Z eine nukleofuge Abgangsgruppe darstellt, mit einem Nukleophil AH, in dem A die für Formel I angegebenen Bedeutungen hat, umsetzt, gegebenenfalls in das reine cis- und trans-Isomere trennt und/oder gegebenenfalls die erhaltene Verbindung in das N-Oxid und/oder in das Säureadditionssalz einer physiologisch verträglichen Säure überführt.

Als nukleofuge Abgangsgruppe für Z kommen Halogenatome, insbesondere Brom oder Chlor, in Betracht.

Die Umsetzung erfolgt zweckmäßig in Gegenwart einer überschüssigen Menge des verwendeten Amins AH, das gleichzeitig als Lösungsmittel und gegebenenfalls als säurebindendes Mittel dient. Gegebenenfalls kann in Gegenwart eines inerten Lösungsmittels, wie einem cyclischen gesättigten Ether, insbesondere Tetrahydrofuran oder Dioxan, von Benzol oder eines Benzolkohlenwasserstoffes, wie Toluol, Xylol, Mesitylen oder Decahydronaphthalin oder eines aprotischen polaren Lösungsmittels, wie Dimethylformamid, gearbeitet werden. Falls nur mit einem Äquivalent des Amins AH gearbeitet wird, muß noch 1 Äquivalent einer inerten Base, wie z.B. Triethylamin, zugesetzt werden.

Die Umsetzung erfolgt in der Regel bei Temperaturen von 80 bis 150°C, und ist im allgemeinen innerhalb von 3 bis 10 Stunden beendet. Gegebenenfalls ist der Ausschluß des Sauerstoffs der Luft und das Arbeiten unter Inertgas, beispielsweise unter Stickstoff, von Vorteil.

Bei den Umsetzungen wird das Nukleophil AH vorteilhafterweise in mindestens 2- bis 20-fachem molarem Überschuß verwendet.

Die Überführung einer Verbindung der Formel I in das N-Oxid erfolgt in üblicher Weise, zweckmäßig mit wäßrigem Wasserstoffperoxid (30 Gew.%) in ethanolischer Lösung. Die Überführung in das Säureadditionssalz einer physiologisch verträglichen Säure erfolgt ebenso in üblicher Weise.

Die Ausgangsverbindungen der Formel II werden erhalten, indem man ein 10-Cyanmethylen-thieno[4,3-e]benzo-4,5-dihydro-azepin-4-on der Formel III

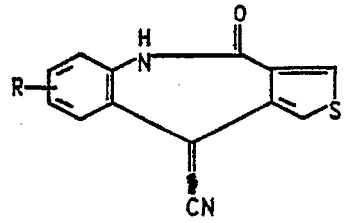

III,

in der

R die für Formel II angegebenen Bedeutungen hat, mit einem Überschuß an Halogenierungsmittel, wie Phosphoroxychlorid, in Gegenwart eines Lösungsmittels und in Gegenwart einer katalytischen Menge N,N-Dimethylanilin 3 bis 5 Stunden auf Rückflußtemperaturen erhitzt und das erhaltene Iminochlorid nach dem Abdestillieren des überchüssigen Phosphoroxychlorids und Aufarbeitung in einem wäßrigen zweiphasigen System durch Extraktion mit einem chlorierten Kohlenwasserstoff, wie Methylenchlorid, isoliert.

Das neue 10-Cyanmethylen-thieno[4,3-e]benzo-4,5-dihydro-azepin-4-on der Formel III, in der R die für Formel I angegebenen Bedeutungen hat, wird durch Carbonyl-Olefinierung hergestellt, indem man ein Thieno[4,3-e]benzo-4,5-dihydro-azepin-4,10-dion der Formel IV

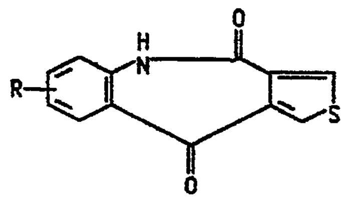
IV,

mit einem Phosphonat der Formel Va

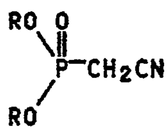
Va,

in der R einen Alkylrest mit 1 bis 3 C-Atomen bedeutet, unter den Bedingungen der Wittig-Horner-Reaktion in einem inerten Lösungsmittel, besonders bevorzugt Dimethylformamid, in Gegenwart von einem Moläquivalent einer Base, bevorzugt Natriumalkoholat, Natriumhydrid oder Natriumamid, bei Temperaturen von 20 bis 80°C umsetzt
oder mit einem Phosphoniumsalz der Formel Vb

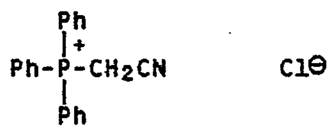
Vb,

in der Ph für einen Phenylrest steht unter den Bedingungen der klassischen Wittig-Reaktion in einem aprotischen organischen Lösungsmittel, insbesondere einem gesättigten aliphatischen oder gesättigten cyclischen Ether, wie Diethylether, Tetrahydrofuran oder Dioxan, oder vorzugsweise in Dimethylformamid, in Gegenwart von einem Moläquivalent einer Base, insbesondere Natriumethylat oder Natriumamid oder einer metallorganischen Verbindung, wie Butyllithium, bei Temperaturen von 20 bis 100°C umsetzt.

Das neue Thieno[4,3-e]benzo-4,5-dihydro-azepin-4,10-dion der Formel IV, in der R die für die Formel I angegebenen Bedeutungen hat, wird durch Friedel-Crafts-Cyclisierung hergestellt, indem man ein Thiophen-3,4-dicarbonsäure-benzamid der Formel VI

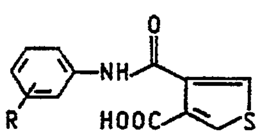
VI,

mit N-Hydroxysuccinimid in Gegenwart eines Moläquivalentes Dicyclohexylcarbodiimid in den aktivierten Ester der Formel VII

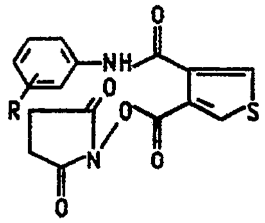

VII,

überführt und diesen dann anschließend in Gegenwart eines 5- bis 8-fachen Überschusses Aluminiumchlorid in einem dipolaren aprotischen Lösungsmittel wie Dimethylformamid nach Friedel-Crafts bei Temperaturen zwischen 50 und 120°C während 1 bis 3 Stunden cyclisiert.

Die erstere Reaktion (VI→VII) wird in einem inerten organischen Lösungsmittel, z.B. Tetrahydrofuran, durchgeführt und ist bei Raumtemperatur innerhalb von 1 bis 3 Stunden beendet. Der ausfallende Harnstoff wird abgesaugt und der aktivierte Ester der Formel VII isoliert.

Das Thiophen-3,4-diocarbonsäure-benzamid der Formel VI erhält man in einfacher Weise durch Umsetzung von Thiophen-3,4-dicarbonsäureanhydrid mit dem entsprechenden Anilin in Tetrahydrofuran bei Raumtemperatur während 1 bis 5 Stunden.

Die erfindungsgemäßen Verbindungen der Formel I werden in der Regel in Form gelblicher bis gelber Kristalle erhalten und können durch Umkristallisation aus den üblichen organischen Lösungsmitteln, bevorzugt aus einem niederen Alkohol, wie Ethanol, umkristallisiert oder durch Säulenchromatographie gereinigt werden.

Falls erforderlich erfolgt eine Trennung in die einzelnen cis- und trans-Isomeren durch fraktionierte Kristallisation in einem chlorierten Kohlenwasserstoff, bevorzugt Methylenchlorid, einem niederen einwertigen Alkohol, bevorzugt Methanol oder Ethanol, oder einem gesättigten cycloaliphatischen Kohlenwasserstoff, bevorzugt Cyclohexan, oder durch Säulenchromatographie, insbesondere in Methylenchlorid und Methanol im Verhältnis 99:1 bis 85:15 Volumenteilen.

Die freien substituierten 10-Cyanmethylen-thieno[4,3-e]benzo-azepine der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure und Zitronensäure.

Die erfindungsgemäßen Verbindungen weisen nach den pharmakologischen Experimenten wertvolle Eigenschaften auf. In Anbetracht ihrer sedativmuskelrelaxierenden, antimonaminergen und zentralanticholinergen Wirkung können sie als Sedativa/Hypnotika, Minor- bzw. Major-Tranquilizer sowie als Antiparkinsonmittel Verwendung finden. Sie eignen sich daher zur Behandlung zentralnervöser Störungen, wie insbesondere Unruhe- und Angstzustände, Schlaflosigkeit, endogene und exogene Psychosen und Parkinsonsche Krankheit.

Mehrere der genannten Wirkungsqualitäten können kombiniert bei einer erfindungsgemäßen Verbindung auftreten. Ein einzelnes Isomeres (nach erfolgter Isomerentrennung) kann eine Wirkung bevorzugt aufweisen.

Zur Wirkungscharakterisierung wurden folgende Methoden angewendet.

1. Sedative Wirkung

4 bis 8 Gruppen von jeweils 3 weiblichen NMRI-Mäusen erhalten die Substanzen oral appliziert. Die photoelektrische Bestimmung der durch eine neue Umgebung induzierten Orientierungshypermotilität erfolgt 30 min nach der Applikation der Substanzen für eine Dauer von 30 min.

Als $ED_{50}$ wird die Dosis bestimmt, welche im Vergleich zu placebobehandelten Kontrolltieren eine Abnahme der Orientierungshypermotilität um 50 % bewirkt.

2. Apomorphinantagonistische Wirkung (Antidopaminerge Wirkung)

Apomorphin (1,21 mg/kg s.c.) führt bei Mäusen, wenn sie in einer geeigneten Umgebung (z.B. Maschendrahtkäfig) gehalten werden, zu einem vermehrten Klettern. Die Tiere werden 30 min lang nach Apomorphingabe beobachtet und das Klettern alle 2 min mit Hilfe eines Score quantifiziert. Die Testsubstanzen werden 60 min vor der Apomorphingabe p.o. gegeben. Als $ED_{50}$ wird die Dosis berechnet, die, im Mittel die bei einem kontrollkollektiv, beobachteten Score-Werte um 50 % vermindert.

3. L-5-HTP-Antagonismus (Serotonin-Antagonismus)

L-5-Hydroxytryptamin (L-5HTP), ein Pro-drug für Serotonin, führt an der Ratte (316 mg/kg i.p.) zu einem Erregungsbild, das durch Symptome wie Kopfschütteln, Tremor und Vorderpfotenbewegungen charakterisiert ist. Die Tiere werden 1 h lang nach L-5HTP-Gabe beobachtet und die auftretenden Symptome

alle 10 min mit Hilfe eines Score quantifiziert. Die Testsubstanzen werden 1 h vor L-5HTP p.o. gegeben. Als $ED_{50}$ wird die Dosis berechnet, die im Mittel die bei einem kontrollkollektiv beobachteten Scorewerte um 50 % reduziert.

4. Anticholinerge Wirkung

Gruppen von 10 weiblichen NMRI-Mäusen erhalten Physostigmin in einer letalen Dosis (0,825 mg/kg) subcutan. Die Prüfsubstanzen werden 30 min vor der Physostigminapplikation oral appliziert. Als $ED_{50}$ wird die Substanzdosis bestimmt, welche 50 % der Tiere vor dem Tod durch Physostigmin schützt.

Die Ergebnisse sind in der Tabelle zusammengestellt.

Tabelle

| Beispiel | Sedative Wirkung | Antimonaminerge Wirkung Apomorphin Antagonismus | L-5HTP | Anticholinerge Wirkung |
|---|---|---|---|---|
| | Maus | Maus | Ratte | Maus |
| | $ED_{50}$ mg/kg p.o. | $ED_{50}$ mg/kg p.o. | | $ED_{50}$ mg/kg p.o. |
| 1 | 0,89 | 1,7 | 0,77 | 15,8 |
| 1 (cis) | 0,35 | 1,0 | 1,0 | >10 |
| 1 (trans) | 5,1 | 10,9 | 33 | 5,2 |
| 9 | 0,81 | 1,0 | | >10 |
| 4 | 3,27 | ~2,0 | 1,0 | ~21 |
| 3a | 0,89 | ~2,0 | 0,8 | >21,5 |
| 16 | ~3,0 | | 1,0 | >10 |
| 5 | 3,75 | ~2,1 | 1,4 | >21,5 |
| Clozapin | 3,8 | 11,3 | 6,3 | 14,1 |

Die Mehrzahl der erfindungsgemäßen Verbindungen zeigen in diesen Experimenten, in denen Sedativa, Minor oder Major Tranquilizer und zentrale Anticholinergica typischerweise wirksam sind, gute Wirkungen. Die Vergleichssubstanz wird in der sedativen und antimonaminergen Wirkung mindestens erreicht, meist jedoch deutlich (bis zum 10fachen) übertroffen. Die anticholinerge Wirkung ist bei der Mehrzahl der Verbindungen geringer ausgeprägt, was bei klinischer Anwendung im Hinblick auf anticholinerge Nebenwirkungen günstig ist.

Die Isomerentrennung zeigt, daß die sedative und antimonaminerge Wirkung bevorzugt beim cis-Isomeren (Beispiel 1) zu finden ist, wobei die anticholinerge Wirkung stark zurücktritt. Das trans-Isomere (Beispiel 1) zeigt dagegen umgekehrt ein Überwiegen der anticholinergen Wirkung bei schwächerer sedativer und antimonaminerger Wirkung. Die anticholinerge Wirkung ist auch stärker als die der Vergleichssubstanzen.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 20 mg/kg Körpergewicht bei oraler Gabe und zwischen 0,1 und 2 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.%.

Die folgenden Beispiele dienen zur Erläuterung der Erfindung:

Beispiel 1

A. Herstellung der Ausgangsstoffe

a) Thiophen-3,4-dicarbonsäure-benzamid

Zu 18,0 g (117 mM) Thiophen-3,4-dicarbonsäureanhydrid in 200 ml Toluol wurden 10,9 g (117 mM) Anilin in 20 ml Toluol bei Raumtemperatur unter gutem Rühren zugetropft. Man ließ anschließend 2 bis 3 h nachrühren, saugte dann die dick ausgefallenen Festkörper ab, wusch gut mit Toluol nach und trocknete die Festkörper zuerst an der Luft und später im Vakuum. Die Ausbeute betrug 28 g (98 %), Schmp. 161-163°C.

b) 4,5-Dihydro-thieno[4,3-e]benzo-azepin-4,10-dion

17,5 g (71 mM) Thiophen-3,4-dicarbonsäurebenzamid wurden in 300 ml Tetrahydrofuran gelöst und unter gutem Rühren mit 8,2 g (71 mM) N-Hydroxysuccinimid sowie 14,7 g (71 mM) N,N'-Dicyclohexyl-carbodiimid versetzt. Man ließ das Reaktionsprodukt 2 h bei Raumtemperatur nachrühren, saugte den ausgefallenen Harnstoff ab, wusch mit wenig Tetrahydrofuran nach und engte das Filtrat zur Trockne ein. Der Rückstand wurde portionsweise in eine Schmelze aus 70 g (526 mM) $AlCl_3$ mit 10,5 ml Dimethylformamid bei 90°C Innentemperatur unter gutem Rühren eingetragen. Man hielt die Reaktionsmischung noch 2 h auf 90°C und goß anschließend den Ansatz noch heiß auf Eis, stellte mit HCl sauer und saugte die hellbraunen Festkörper nach einigem Rühren ab. Nach dem Nachwaschen mit $H_2O$ wurde das Produkt an der Luft getrocknet. Man erhielt 15,3 g (94 %) Produkt mit genügender Reinheit für die weitere Umsetzung.

c) cis,trans-10-Cyanmethylen-4,5-dihydro-thieno[4,3-e]benzo-azepin-4-on
(cis,trans-Isomerengemisch)

Zur Herstellung dieses Produktes führte man eine Carbonyl-Olefinierung des 4,5-Dihydro-thieno[4,3-e]benzo-azepin-4,10-dions mit Hilfe der Wittig-Horner-Reaktion (a) oder über die klassische Wittig-Synthese (b) durch:

a) 8,8 g (38,5 mM) 4,5-Dihydro-thieno[4,3-e]benzo-azepin-4,10-dion wurden in 70 ml Dimethylformamid unter Erwärmen gelöst und unter Stickstoff gerührt. Man ließ dann gleichzeitig 8,0 g (45 mM) Diethyl-cyanmethyl-phosphonat und 8,1 g (45 mM) Natriummethylat (30 %) gelöst in 10 ml Dimethylformamid langsam zutropfen (Farbzunahme und Temperaturerhöhung zeigen den Beginn der Wittig-Reaktion an). Nach 12 h Nachrühren bei Raumtemperatur goß man das Reaktionsprodukt auf Eiswasser und saugte die ausfallenden Festkörper ab. Nach gutem Nachwaschen mit Wasser wurde das Rohprodukt getrocknet und aus Ethanol umkristallisiert. Ausbeute: 8,8 g (91 %) 10-Cyanmethylen-4,5-dihydro-thieno;[4,3-e]benzo-azepin-4-on, farblose Kristalle, Schmp. >265°C.
b) Man legte Triphenyl-cyanomethyl-phosphonium-chlorid in Dimethylformamid vor, ließ anschließend 1 Moläquivalent einer 30 %igen Natriummethylat-Lösung zutropfen oder versetzte mit 1 Moläquivalent Natriumhydrid und fügte zuletzt 1 Moläquivalent einer Lösung von 4,5-Dihydro-thieno[4,3-e]benzo-azepin-4,10-dion in Dimethylformamid hinzu. Man ließ das Reaktionsgemisch 5 bis 8 h bei 50 bis 80°C nachrühren, goß anschließend auf Eiswasser und extrahierte mehrmals mit Methylenchlorid. Nach dem Trocknen und Einengen der organischen Phase kristallisierte man das Rohprodukt aus Ethanol um. Ausbeute: 65 % farblose Kristalle, Schmp. >265°C.

B. Herstellung des Endprodukts

cis- und trans-10-Cyanmethylen-4-(4-methyl-piperazin-1-yl)-thieno[4,3-e]benzo-azepin

a) 11,0 g (44 mM) 10-Cyanmethylen-4,5-dihydro-thieno[4,3-e]4,3-e]benzo-azepin-4-on (cis,trans-Isomerengemisch) in 100 ml 1,1,2-Trichlorethan wurden mit 30 ml Phosphoroxychlorid und 1,0 ml N,N-Dimethylanilin versetzt und 0,5 h unter Stickstoffatmosphäre unter Rückfluß gekocht. Nach vollständigem Abdestillieren des überschüssigen Phosphoroxychlorids und Dimethylanilins im Ölpumpenvakuum verteilte man den Rückstand zwischen Methylenchlorid und Wasser, extrahierte die wäßrige Phase noch zweimal mit Methylenchlorid und wusch die vereinigten organischen Phasen mit verdünnter HCl und Wasser gründlich nach. Trocknen und Einengen der organischen Phase lieferte 11,6 g (98 %) 4-Chlor-10-cyanmethylen-thieno[4,3-e]benzo-azepin, das für die weitere Umsetzung genügend rein ist.
11,6 g (43 mM) 4-Chlor-10-cyanmethylen-thieno[4,3-e]benzo-azepin wurden in 70 ml Dimethylformamid gelöst, mit 10 ml (90 mM) N-Methyl-piperazin und 10 ml (75 mM) Triethylamin (stark exotherme Reaktion) versetzt und 2 bis 3 h bei 100°C unter Stickstoff gerührt. Das dunkle homogene Reaktionsgemisch goß man dann nach dem Abkühlen auf Eiswasser und saugte das gelbliche Rohprodukt, 10-Cyanmethylen-4-(4-methyl-piperazin-1-yl)-thieno[4,3-e]benzo-azepin, ab. Nach dem Trocknen im Vakuumtrockenschrank kristallisierte man das Rohprodukt aus Ethanol unter Zusatz von Aktivkohle um oder reinigte durch Säulenchromatographie (Kieselgel, Laufmittel Methylenchlorid/Methanol 95/5). Man erhielt 11,6 g (81 %) gelbliches 10-Cyanmethylen-4-(4-methyl-piperazin-1-yl)-thieno[4,3-e]benzo-azepin in Form eines cis,trans-Isomerengemisches, Schmp. 90 bis 92°C.

b) Zur Trennung der cis,trans-Isomeren kristallisierte man das Isomerengemisch fraktioniert aus Ethanol um. Man isolierte als erste Fraktion 3,1 g gelbe Kristalle die nach dem Dünnschichtchromatogramm (Kieselgel, Laufmittel Toluol/Methanol 85/15) vornehmlich aus dem unpolaren Isomeren a bestehen.

Aus dem Filtrat kristallisierten, nachdem man die Lösung durch Einengen etwas konzentriert hatte, langsam 2,9 g gelbe Kristalle, die nach dem Dünnschichtchromatogramm (Kieselgel, Laufmittel Toluol/Methanol 85/15) vornehmlich aus dem polaren Isomeren b bestehen.

Durch darauffolgende Kristallisation der angereicherten Produkte a und b aus Ethanol fielen die cis,trans-Isomeren praktisch rein an.

Die Röntgenstrukturanalyse zeigte, daß es sich bei a um das cis-isomere und bei b um das trans-isomere 10-Cyanmethylen-4-(4-methyl-piperazin-1-yl)-thieno[4,3-e]benzo-azepin handelt.

Schmp.: Cis-Isomeres a 191-193°C, trans-Isomeres b 220-221°C.

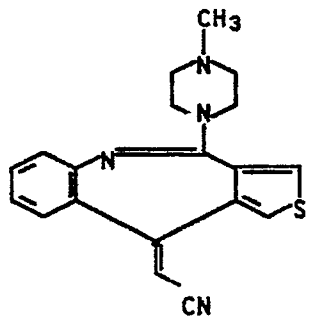

a

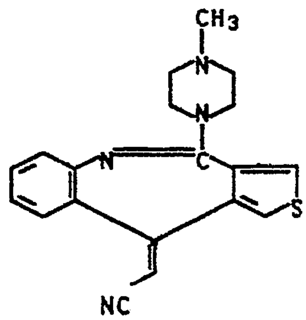

b

Beispiel 2

cis,trans-10-Cyanmethylen-4-(4-methyl-4-oxy-piperazin-1-yl)-thieno[4,3-e]benzo-azepin

2,8 g (8,4 mM) cis,trans-10-Cyanmethylen-4-(4-methyl-piperazin-1-yl)-thieno [4,3-e]benzo-azepin (vgl. Beispiel 1) wurden in 100 ml heißem Ethanol gelöst und mit 1,5 ml 30 %igem Wasserstoffperoxid versetzt. Nach 5 h Rückflußkochen zerstörte man das überschüssige Wasserstoffperoxid mit Hilfe eines kleinen Platinbleches, das in das Reaktionsgemisch geworfen wurde, durch 2 h Rückflußkochen. Nach dem Filtrieren engte man das Reaktionsgemisch ein und reinigte das erhaltene N-Oxid durch Säulenchromatographie (Kieselgel, Laufmittel Methylenchlorid/Methanol 95/5). Man isolierte 1,8 g (63 %) gelbe Kristalle.

Die folgenden Substanzen erhält man analog Beispiel 1 und 2 unter Einsatz der entsprechenden substituierten Amine:

3. cis,trans-7-Chlor-10-cyanmethylen-4-(4-methyl-piperazin-1-yl)-thieno[4,3-e]benzo-azepin.
3a. cis-7-Chlor-10-cyanmethylen-4-(4-methyl-piperazin-1-yl)-thieno[4,3-e]benzo-azepin.
3b. trans-7-Chlor-10-cyanmethylen-4-(4-methyl-piperazin-1-yl)-thieno[4,3-e]benzo-azepin.
4. cis,trans-7-Fluor-10-cyanmethylen-4-(4-methyl-piperazin-1-yl)-thieno[4,3-e]benzo-azepin.
5. cis,trans-7-Methyl-10-cyanmethylen-4-(4-methyl-piperazin-1-yl)-thieno[4,3-e]benzo-azepin.
6. cis,trans-7-Trifluormethyl-10-cyanmethylen-4-(4-methyl-piperazin-1-yl)-thieno[4,3-e]benzo-azepin.
7. cis,trans-7-Methoxy-10-cyanmethylen-4-(4-methyl-piperazin-1-yl)-thieno[4,3-e]benzo-azepin.
8. cis,trans-10-Cyanmethylen-4-(piperazin-1-yl)-thieno[4,3-e]benzo-azepin.
9. cis,trans-10-Cyanmethylen-4-(4-ethyl-piperazin-1-yl)-thieno[4,3-e]benzo-azepin.
10. cis,trans-10-Cyanmethylen-4-(N'-methyl-homopiperazin-1-yl)-thieno[4,3-e]benzo-azepin.
11. cis,trans-10-Cyanmethylen-4-(2-piperidin-1-yl)-ethyl-amino)-thieno[4,3-e]benzo-azepin.
12. cis,trans-10-Cyanmethylen-4-(2-dimethylamino-ethylamino)-thieno[4,3-e]benzo-azepin.
13. cis,trans-10-Cyanmethylen-4-(4-cyclopropyl-piperazin-1-yl)-thieno[4,3-e]benzo-azepin.
14. cis,trans-10-Cyanmethylen-4-(4-cyclopropylmethyl-piperazin-1-yl)-thieno[4,3-e]benzo-azepin.
15. cis,trans-10-Cyanmethylen-4-(4-propin-2-yl-piperazin-1-yl)-thieno[4,3-e]benzo-azepin.
16. trans-6-Chlor-10-cyanmethylen-4-(4-methyl-piperazin-1-yl)-thieno-[4,3-e]benzo-azepin

Beispiel 19

Auf einer Tablettenpresse werden in üblicher Weise Tabletten folgender Zusammensetzung gepreßt:
40 mg Substanz des Beispiels 1 (cis)
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6 %iger Kleister)

Beispiel 20

In üblicher Weise werden Dragees folgender Zusammensetzung hergestellt:
20 mg Substanz des Beispiels 1 (cis)
60 mg Kernmasse
60 mg Verzuckerungsmasse
Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 21

10 g Substanz des Beispiels 1 (cis) werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

**Patentansprüche für die Vertragsstaaten: BE, CH, DE, FR, GB, IT, NL, SE**

1. 4-substituierte 10-Cyanmethylen-thieno[4,3-e]benzo-azepine der Formel I

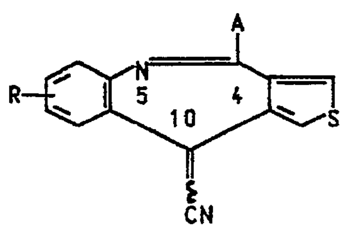

I,

in der
R ein Wasserstoff- oder Halogenatom, einen Alkylrest mit 1 bis 3 C-Atomen, Trifluormethyl oder einen Alkoxyrest mit 1 bis 3 C-Atomen bedeutet, und A für einen Aminorest -$NR^1R^2$ steht, in dem $R^1$ und $R^2$ zusammen mit dem sie verbindenden Stickstoffatom einen 5- bis 7-gliedrigen gesättigten Ring bedeuten, der gegebenenfalls als weiteres Heteroatom ein Stickstoff- oder Sauerstoffatom enthält, wobei ein zusätzlich vorhandenes Stickstoffatom gegebenenfalls durch einen Alkylrest mit 1 bis 3 C-Atomen, einen Hydroxyalkylrest mit 2 bis 3 C-Atomen, einen Alkoxyalkylrest mit 1 bis 3 C-Atomen im Alkyl- und Alkoxyrest, einen Cycloalkyl- oder Cycloalkylmethylrest mit 3 bis 7 C-Atomen im Cycloalkylring, einen Alkinylrest mit 2 bis 5 C-Atomen und gegebenenfalls zusätzlich durch Sauerstoff in Form eines N-Oxids substituiert ist, oder A einen Aminorest -$NHR^3$, in dem $R^3$ einen Aminoalkylrest mit 2 bis 7 C-Atomen bedeutet, wobei das Aminstickstoffatom gegebenenfalls durch einen niederen Alkylrest mit 1 bis 5 C-Atomen substituiert oder Bestandteil eines 5- bis 7-gliedrigen gesättigten Ringes ist, der gegebenenfalls ein Stickstoff- oder Sauerstoffatom als weiteres Heteroatom enthält, wobei ein vorhandenes Stickstoffatom durch einen niederen Alkylrest mit 1 bis 3 C-Atomen oder einen Hydroxyalkylrest mit 2 bis 3 C-Atomen substituiert sein kann, bedeutet, und ihre physiologisch verträglichen Säureadditionssalze.

2. Verbindungen der Formel I nach Anspruch 1, in denen R Wasserstoff, Chlor oder Fluor bedeutet und A für Piperidin, Piperazin oder Homopiperazin steht, die am gegebenenfalls vorhandenen Ringstickstoffatom durch Wasserstoff, Methyl, Ethyl, β-Hydroxyethyl, Cyclopropyl oder Propinyl substituiert sind und/oder gegebenenfalls in Form des N-Oxids vorliegen können.

3. cis,trans-10-Cyanmethylen-4-(4-methyl-piperazin-1-yl)-thieno[4,3-e]benzo-azepin.

4. cis-10-Cyanmethylen-4-(4-methyl-piperazin-1-yl)-thieno[4,3-e]benzo-azepin.

5. trans-10-Cyanmethylen-4-(4-methyl-piperazin-1-yl)-thieno[4,3-e]benzo-azepin.

6. cis,trans-7-Chlor-10-cyanmethylen-4-(4-methyl-piperazin-1-yl)-thieno[4,3-e]benzo-azepin.

7. cis-7-Chlor-10-cyanmethylen-4-(4-methyl-piperazin-1-yl)-thieno[4,3-e]benzo-azepin.

8. trans-7-Chlor-10-cyanmethylen-4-(4-methyl-piperazin-1-yl)-thieno[4,3-e]benzo-azepin.

9. cis,trans-7-Fluor-10-cyanmethylen-4-(4-methyl-piperazin-1-yl)-thieno[4,3-e]benzo-azepin.

10. Verfahren zur Herstellung von Verbindungen der Formel I, dadurch gekennzeichnet, daß man eine Verbindung der Formel II

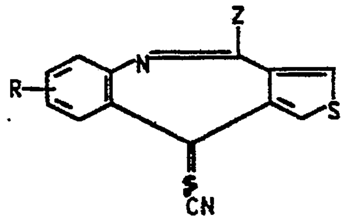

II,

in der R die für Formel I gemäß Anspruch 1 angegebenen Bedeutungen hat und Z eine nukleofuge Abgangsgruppe darstellt, mit einem Amin AH, in dem A die für Formel I angegebenen Bedeutungen hat, umsetzt und gegebenenfalls die erhaltene Verbindung in das N-Oxid und/oder in das Säureadditionssalz einer physiologisch verträglichen Säure überführt.

11. Therapeutisches Mittel, gekennzeichnet durch einen Gehalt an einer Verbindung der Formel I gemäß Anspruch 1 oder deren pharmakologisch verträgliches Säureadditionssalz als Wirkstoff neben üblichen Träger- und Verdünnungsmitteln.

12. Verbindungen der Formel I gemäß Anspruch 1 zur Verwendung bei der Bekämpfung von Krankheiten.

**Patentansprüche für den Vertragsstaat: AT**

1. Verfahren zur Herstellung der 4-substituierte 10-Cyanmethylen-thieno[4,3-e]benzo-azepine der Formel I

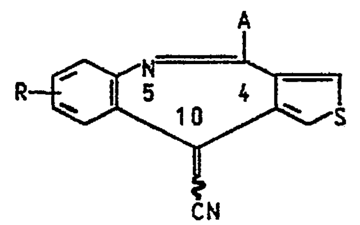

I,

in der
R ein Wasserstoff- oder Halogenatom, einen Alkylrest mit 1 bis 3 C-Atomen, Trifluormethyl oder einen Alkoxyrest mit 1 bis 3 C-Atomen bedeutet, und A für einen Aminorest $-NR^1R^2$ steht, in dem $R^1$ und $R^2$ zusammen mit dem sie verbindenden Stickstoffatom einen 5- bis 7-gliedrigen gesättigten Ring bedeuten, der gegebenenfalls als weiteres Heteroatom ein Stickstoff- oder Sauerstoffatom enthält, wobei ein zusätzlich vorhandenes Stickstoffatom gegebenenfalls durch einen Alkylrest mit 1 bis 3 C-Atomen, einen Hydroxyalkylrest mit 2 bis 3 C-Atomen, einen Alkoxyalkylrest mit 1 bis 3 C-Atomen im Alkyl- und Alkoxyrest, einen Cycloalkyl- oder Cycloalkylmethylrest mit 3 bis 7 C-Atomen im Cycloalkylring, einen Alkinylrest mit 2 bis 5 C-Atomen und gegebenenfalls zusätzlich durch Sauerstoff in Form eines N-Oxids substituiert ist, oder A einen Aminorest $-NHR^3$, in dem $R^3$ einen Aminoalkylrest mit 2 bis 7 C-Atomen bedeutet, wobei das Aminstickstoffatom gegebenenfalls durch einen niederen Alkylrest mit 1 bis 5 C-Atomen substituiert oder Bestandteil eines 5- bis 7-gliedrigen gesättigten Ringes ist, der gegebenenfalls ein Stickstoff- oder Sauerstoffatom als weiteres Heteroatom enthält, wobei ein vorhandenes Stickstoffatom durch einen niederen Alkylrest mit 1 bis 3 C-Atomen oder einen Hydroxyalkylrest mit 2 bis 3 C-Atomen substituiert sein kann, bedeutet, und ihre physiologisch verträglichen Säureadditionssalze, dadurch gekennzeichnet, daß man eine Verbindung der Formel II

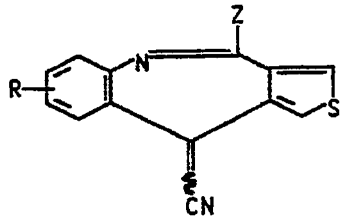

II,

in der R die für Formel I angegebenen Bedeutungen hat und Z eine nukleofuge Abgangsgruppe darstellt, mit einem Amin AH, in dem A die für Formel I angegebenen Bedeutungen hat, umsetzt und gegebenenfalls die erhaltene Verbindung in das N-Oxid und/oder in das Säureadditionssalz einer physiologisch verträglichen Säure überführt.

2. Verfahren gemäß Anspruch 1, dadurch gekennzeichnet, daß man Verbindungen der Formel I herstellt, in denen R Wasserstoff, Chlor oder Fluor bedeutet und A für Piperidin, Piperazin oder Homopiperazin steht, die am gegebenenfalls vorhandenen Ringstickstoffatom durch Wasserstoff, Methyl, Ethyl, β-Hydroxyethyl, Cyclopropyl oder Propinyl substituiert sind und/oder gegebenenfalls in Form des N-Oxids vorliegen können.

3. Verfahren gemäß Anspruch 1, dadurch gekennzeichnet, daß man cis,trans-10-Cyanmethylen-4-(4-methyl-piperazin-1-yl)-thieno[4,3-e]benzo-azepin herstellt.

4. Verfahren gemäß Anspruch 1, dadurch gekennzeichnet, daß man cis-10-Cyanmethylen-4-(4-methyl-piperazin-1-yl)-thieno[4,3-e]benzo-azepin herstellt.

5. Verfahren gemäß Anspruch 1, dadurch gekennzeichnet, daß man trans-10-Cyanmethylen-4-(4-methyl-piperazin-1-yl)-thieno[4,3-e]benzo-azepin herstellt.

6. Verfahren gemäß Anspruch 1, dadurch gekennzeichnet, daß man cis,trans-7-Chlor-10-cyanmethylen-(4-methyl-piperazin-1-yl)-thieno[4,3-e]benzo-azepin herstellt.

7. Verfahren gemäß Anspruch 1, dadurch gekennzeichnet, daß man cis-7-Chlor-10-cyanmethylen-4-(4-methyl-piperazin-1-yl)-thieno[4,3-e]benzo-azepin herstellt.

8. Verfahren gemäß Anspruch 1, dadurch gekennzeichnet, daß man trans-7-Chlor-10-cyanmethylen-4-(4-methyl-piperazin-1-yl)-thieno[4,3-e]benzo-azepin herstellt.

9. Verfahren gemäß Anspruch 1, dadurch gekennzeichnet, daß man cis,trans-7-Fluor-10-cyanmethylen-4-(4-methyl-piperazin-1-yl)-thieno[4,3-e]benzo-azepin herstellt.

9. Verfahren gemäß Anspruch 1, dadurch gekennzeichnet, daß man cis,trans-7-Fluor-10-cyanmethylen-4-(4-methyl-piperazin-1-yl)-thieno[4,3-e]benzo-azepin herstellt.

**Claims for the Contracting States: BE, CH, DE, FR, GB, IT, NL, SE**

1. A 4-substituted 10-cyanomethylenethieno[4,3-e]-benzoazepine of the formula I

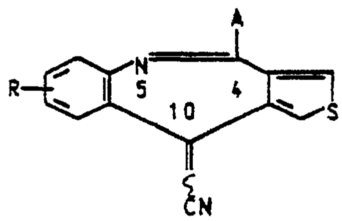

I

where R is hydrogen, halogen, alkyl of 1 to 3 carbon atoms, trifluoromethyl or alkoxy of 1 to 3 carbon atoms and A is an amino radical $-NR^1R^2$, in which $R^1$ and $R^2$, together with the nitrogen atom to which they are bonded, form a saturated 5-membered, 6-membered or 7-membered ring which may contain nitrogen or oxygen as a further heteroatom, and any additional nitrogen atom present is unsubstituted or substituted by alkyl of 1 to 3 carbon atoms, hydroxyalkyl of 2 or 3 carbon atoms, alkoxyalkyl where the alkyl and alkoxy radicals are each of 1 to 3 carbon atoms, cycloalkyl or cycloalkylmethyl, each of which has 3 to 7 carbon atoms in the cycloalkyl ring, or alkynyl of 2 to 5 carbon atoms, and may additonally be substituted by oxygen in the form of an N-oxide, or A is an amino radical $-NHR^3$ where $R^3$ is aminoalkyl of 2 to 7 carbon atoms and the amine nitrogen is unsubstituted or substituted by lower alkyl of 1 to 5 carbon atoms or forms part of a saturated 5-membered, 6-membered or 7-membered ring which may contain nitrogen or oxygen as a further heteroatom, and any nitrogen atom present may be substituted by lower alkyl of 1 to 3 carbon atoms or hydroxyalkyl of 2 or 3 carbon atoms, and its physiologically tolerated addition salts with acids.

2. A compound of the formula I as claimed in claim 1, wherein R is hydrogen, chlorine or fluorine and A is piperidinyl, piperazinyl or homopiperazinyl, which are substituted at any ring nitrogen atom which may be present by hydrogen, methyl, ethyl, β-hydroxyethyl, cyclopropyl or propynyl and/or may be present in the form of the N-oxide.

3. cis,trans-10-Cyanomethylene-4-(4-methylpiperazin-1-yl)-thieno[4,3-e]benzoazepine.

4. cis-10-Cyanomethylene-4-(4-methylpiperazin-1-yl)-thieno[4,3-e]benzoazepine.

5. trans-10-Cyanomethylene-4-(4-methylpiperazin-1-yl)-thieno[4,3-e]benzoazepine.

6. cis,trans-7-Chloro-10-cyanomethylene-4-(4-methyl-piperazin-1-yl)-thieno[4,3-e]benzoazepine.

7. cis-7-Chloro-10-cyanomethylene-4-(4-methylpiperazin-1-yl)-thieno[4,3-e]benzoazepine.

8. trans-7-Chloro-10-cyanomethylene-4-(4-methyl-piperazin-1-yl)-thieno[4,3-e]benzoazepine.

9. cis,trans-7-Fluoro-10-cyanomethylene-4-(4-methyl-piperazin-1-yl)-tienol[4,3-e]benzoazepine.

10. A process for preparing a compound of the formula I, wherein a compound of the formula II

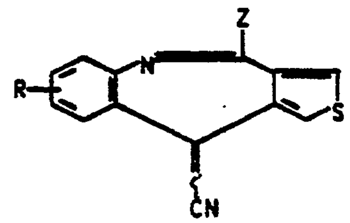

II

where R has the meanings given for formula I as claimed in claim 1 and Z is a nucleofugic leaving group, is reacted with an amine AH, where A has the meanings given for formula I, and, if desired, the resulting compound is converted to the N-oxide or to an addition salt with a physiologically tolerated acid.

11. A therapeutic agent which contains a compound of the formula I as claimed in claim 1 or a pharmacologically acceptable acid addition salt thereof as an acitve compound, as well as conventional cariers and diluents.

12. A compound of the formula I as claimed in claim 1 for use in the treatment of disorders.

**Claims for the Contracting State: AT**

1. A process for preparing a 4-substituted 10-cyanomethylenethieno[4,3-e]benzoazepine of the formula I

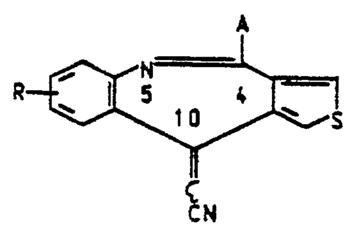

I

where R is hydrogen, halogen, alkyl of 1 to 3 carbon atoms, trifluoromethyl or alkoxy of 1 to 3 carbon atoms and A is an amino radical $-NR^1R^2$, in which $R^1$ and $R^2$, together with the nitrogen atom to which they are bonded, form a saturated 5-membered, 6-membered or 7-membered ring which may contain nitrogen or oxygen as a further heteroatom, and any additional nitrogen atom present is unsubstituted or substituted by alkyl of 1 to 3 carbon atoms, hydroxyalkyl of 2 or 3 carbon atoms, alkoxyalkyl where the alkyl and alkoxy radicals are each of 1 to 3 carbon atoms, cycloalkyl or cycloalkylmethyl, each of which as 3 to 7 carbon atoms in the cycloalkyl ring, or alkynyl of 2 to 5 carbon atoms, and may additionally be substituted by oxygen in the form of an N-oxide, or A is an amino radical $-NHR^3$ where $R^3$ is aminoalkyl of 2 to 7 carbon atoms and the amine nitrogen is unsubstituted or substituted by lower alkyl of 1 to 5 carbon atoms for forms part of a saturated 5-membered, 6-membered or 7-membered ring which may contain nitrogen or oxygen as a further heteroatom, and any nitrogen atom present may be substituted by lower alkyl of 1 to 3 carbon atoms or hydroxyalkyl of 2 or 3 carbon atoms, and its physiologically tolerated addition salts with acids wherein a compound of the formula II

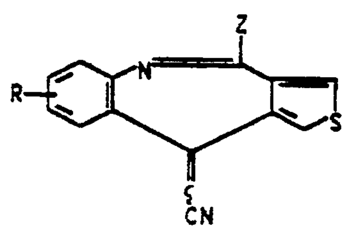

II

where R has the meanings given for formula I and Z is a nucleofugic leaving group, is reacted with an amine AH, where A has the meanings given for formula I, and, if desired, the resulting compound is converted to the N-oxide or to an addition salt with a physiologically tolerated acid.

2. A process as claimed in claim 1, wherein a compound of the formula I is prepared in which R is hydrogen, chlorine or fluorine, A is piperidinyl, piperazinyl or homopiperazinyl, which are substituted on any ring nitrogen atom which may be present by hydrogen, methyl, ethyl, β-hydroxyethyl, cyclopropyl or propynyl and/or may be present in the form of the N-oxide.

3. A process as claimed in claim 1, wherein cis, trans-10-cyanomethylene-4-(4-methylpiperazin-1-yl)-thieno[4,3-e]benzoazepine is prepared.

4. A process as claimed in claim 1, wherein cis-10-cyanomethylene-4-(4-methylpiperazin-1-yl)-thieno[4,3-e]benzoazepine is prepared.

5. A process as claimed in claim 1, wherein trans-10-cyanomethylene-4-(4-methylpiperazin-1-yl)-thieno[4,3-e]benzoazepine is prepared.

6. A process as claimed in claim 1, wherein cis, trans-7-chloro-10-cyanomethylene-4-(4-methylpiperazin-1-yl)-thieno[4,3-e]benzoazepine is prepared.

7. A process as claimed in claim 1, wherein cis-7-chloro-10-cyanomethylene-4-(4-methylpiperazin-1-yl)-thieno[4,3-e]benzoazepine is prepared.

8. A process as claimed in claim 1, wherein trans-7-chloro-10-cyanomethylene-4-(4-methylpiperazin-1-yl)-thieno[4,3-e]benzoazepine is prepared.

9. A process as claimed in claim 1, wherein cis-trans-7-fluoro-10-cyanomethylene-4-(4-methylpiperazin-1-yl)-thieno[4,3-e]benzoazepine is prepared.

**Revendications pour les Etats contractants: BE, CH, DE, FR, GB, IT, NL, SE**

1. 10-cyanométhylen-thiéno[4,3-e]benzo-azépines substituées en 4 de formule I

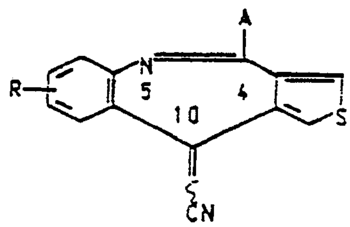

I,

dans laquelle
R représente un atome d'hydrogène ou d'halogène, un reste alkyle ayant 1 à 3 atomes C, trifluorométhyle ou un reste alcoxy ayant 1 à 3 atomes C et A est mis pour un reste amino $-NR^1R^2$, dans lequel $R^1$ et $R^2$ ensemble avec l'atome d'azote qui les relie représentent un noyau saturé ayant 5 à 7 chaînons, qui contient éventuellement, comme autre hétéroatome, un atome d'azote ou d'oxygène, un atome d'azote présent additionnellement étant substitué éventuellement par un reste alkyle ayant 1 à 3 atomes C, un reste hydroxyalkyle ayant 2 à 3 atomes C, un reste alcoxyalkyle ayant 1 à 3 atomes C dans le reste alkyle et alcoxy, un reste cycloalkyle ou cycloalkylméthyle ayant 3 à 7 atomes C dans le noyau cycloalkyle, un reste alcynyle ayant 2 à 5 atomes C et étant éventuellement substitué additionnellement par de l'oxygène sous forme d'un oxyde-N, ou A représente un reste amino $-NHR^3$, dans lequel $R^3$ représente un reste aminoalkyle ayant 2 à 7 atomes C, l'atome azote d'amine étant éventuellement subsitué par un reste alkyle inférieur ayant 1 à 5 atomes C ou bien est composant d'un noyau saturé de 5 à 7 chaînons, qui contient éventuellement, comme autre hétéro-atome, un atome d'azote ou d'oxygène, un atome d'azote présent pouvant être substitué par un reste alkyle inférieur ayant 1 à 3 atomes C ou un reste hydroxyalkyle ayant 2 à 3 atomes C, ou leurs sels d'addition d'acide tolérables physiologiquement.

2. Composés de formule I selon la revendication 1, dans lesquels R représente hydrogène, chlore ou fluor et A est mis pour pipéridine, pipérazine ou homopipérazine, qui, sur l'atome d'azote du noyau éventuellement présent, sont substitués par hydrogène, méthyle, éthyle, β-hydroxyéthyle, cyclopropyle ou propinyle et/ou peuvent se trouver éventuellement sous forme de l'oxyde-N.

3. cis, trans-10-cyanométhylen-4-(4-méthyl-pipérazin-1-yl)-thiéno[4,3-e]-benzo-azépine.

4. cis-10-cyanométhylen-4-(4-méthyl-pipérazin-1-yl)-thiéno[4,3-e]benzo-azépine.

5. trans-10-cyanométhylen-4-(4-méthyl-pipérazin-1-yl)-thiéno[4,3-e]benzo-azépine.

6. cis, trans-7-chloro-10-cyanométhylen-4-(4-méthyl-pipérazin-1-yl)-thiéno[4,3-e]benzo-azépine.

7. cis-7-chloro-10-cyanométhylen-4-(4-méthyl-pipérazin-1-yl)-thiéno-[4,3-e]benzo-azépine.

8. trans-7-chloro-10-cyanométhylen-4-(4-méthyl-pipérazin-1-yl)-thiéno[4,3-e]benzo-azépine.

9. cis, trans-7-fluoro-10-cyanométhylen-4-(4-méthyl-pipérazin-1-yl)-thiéno[4,3-e]benzo-azépine.

10. Procédé de préparation de composés de formule I, caractérisé par le fait que l'on fait réagir un composé de formule II

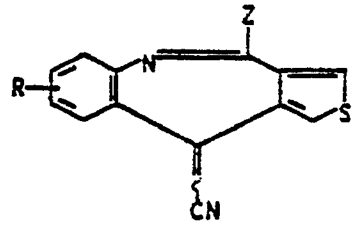

II.

dans laquelle R a les significations données pour la formule I selon la revendication 1 et Z représente un groupe éliminable nucléofuge, avec une amine AH, où A a les significations données pour la formule I, et on transforme éventuellement le composé obtenu en l'oxyde-N et/ou en le sel d'addition d'un acide tolérable physiologiquement.

11. Agent thérapeutique, caractérisé par le fait qu'il contient un composé de formule I selon la revendication 1 ou son sel d'addition d'acide tolérable pharmacologiquement, comme principe actif à côté de supports et diluants usuels.

12. Composés de formule I selon la revendication 1 pour utilisation dans la lutte contre les maladies.

**Revendications pour l'Etat contractant: AT**

1. Procédé de préparation des 10-cyanométhylen-thiéno[4,3-e]benzo-azépines substituées en 4 de formule I

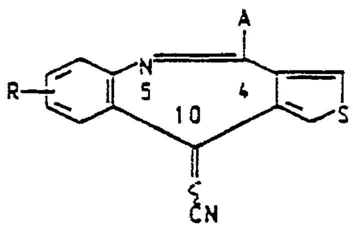

I,

dans laquelle
R représente un atome d'hydrogène ou d'halogène, un reste alkyle ayant 1 à 3 atomes C, trifluorométhyle ou un reste alcoxy ayant 1 à 3 atomes C et A est mis pour un reste amino $-NR^1R^2$, dans lequel $R^1$ et $R^2$ ensemble avec l'atome d'azote qui les relie représentent un noyau saturé ayant 5 à 7 chaînons, qui contient éventuellement, comme autre hétéroatome, un atome d'azote ou d'oxygène, un atome d'azote présent additionnellement étant substitué éventuellement par un reste alkyle ayant 1 à 3 atomes C, un reste hydroxyalkyle ayant 2 à 3 atomes C, un reste alcoxyalkyle ayant 1 à 3 atomes C dans le reste alkyle et alcoxy, un reste cycloalkyle ou cycloalkylméthyle ayant 3 à 7 atomes C dans le noyau cycloalkyle, un reste alcynyle ayant 2 à 5 atomes C et étant éventuellement substitué additionnellement par de l'oxygène sous forme d'un oxyde-N, ou A représente un reste amino $-NHR^3$, dans lequel $R^3$ représente un reste aminoalkyle ayant 2 à 7 atomes C, l'atome azote d'amine étant éventuellement substitué par un reste alkyle inférieur ayant 1 à 5 atomes C ou bien est composant d'un noyau saturé de 5 à 7 chaînons, qui contient éventuellement, comme autre hétéro-atome, un atome d'azote ou d'oxygène, un atome d'azote présent pouvant être substitué par un reste alkyle inférieur ayant 1 à 3 atomes C ou un reste hydroxyalkyle ayant 2 à 3 atomes C, ou leurs sels d'addition d'acide tolérables physiologiquement, caractérisé par le fait que l'on fait réagir un composé de formule II

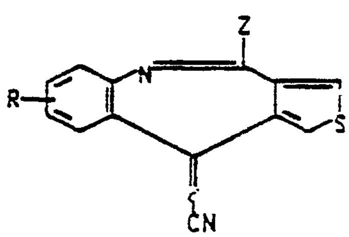

II.

dans laquelle R a les significations données pour la formule I selon la revendication 1 et Z représente un groupe éliminable nucléofuge, avec une amine AH, où A a les significations données pour la formule I, et on transforme éventuellement le composé obtenu en l'oxyde-N et/ou en le sel d'addition d'un acide tolérable physiologiquement.

2. Procédé selon la revendication 1, caractérisé par le fait qu'on fait réagir des composés de formule

I, dans lesquels R représente hydrogène, chlore ou fluor et A est mis pour pipéridine, pipérazine ou homopiperazine, qui, sur l'atome d'azote du noyau éventuellement présent, sont substitués par hydrogène, méthyle, éthyle, β-hydroxyéthyle, cyclopropyle ou propinyle et/ou peuvent se trouver éventuellement sous forme de l'oxyde-N.

3. Procédé selon la revendication 1, caractérisé par le fait qu'on prépare de la cis-,trans-10-cyanométhylen-4-(4-méthyl-pipérazin-1-yl)-thiéno[4,3-e]benzo-azépine.

4. Procédé selon la revendication 1, caractérisé par le fait qu'on prépare de la cis-10-cyanométhylen-4-(4-méthyl-pipérazin-1-yl)-thiéno[4,3-e]benzo-azépine.

5. Procédé selon la revendication 1, caractérisé par le fait qu'on prépare de la trans-10-cyanométhylen-4-(4-méthyl-pipérazin-1-yl)-thiéno[4,3-e]benzo-azépine.

6. Procédé selon la revendication 1, caractérisé par le fait qu'on prépare de la cis-,trans-7-chloro-10-cyanométhylen-4-(4-méthyl-pipérazin-1-yl)-thiéno-[4,3-e]benzo-azépine.

7. Procédé selon la revendication 1, caractérisé par le fait qu'on prépare de la cis-7-chloro-10-cyanométhylen-4-(4-méthyl-pipérazin-1-yl)-thiéno-[4,3-e]benzo-azépine.

8. Procédé selon la revendication 1, caractérisé par le fait qu'on prépare de la trans-7-chloro-10-cyanométhylen-4-(4-méthyl-pipérazin-1-yl)-thiéno-[4,3-e]benzo-azépine.

9. Procédé selon la revendication 1, caractérisé par le fait qu'on prépare de la cis-, trans-7-fluoro-10-cyanométhylen-4-(4-méthyl-pipérazin-1-yl)-thiéno-[4,3-e]benzo-azépine.